# EUROPEAN PATENT APPLICATION

(11) **EP 0 899 343 A2**
(43) Date of publication of application: **03.03.1999**
(21) Application number: 98305933.8
(22) Date of filing: 24.07.1998
(51) Int. Cl.: C12P 19/04, C07K 16/12, A61K 39/106, C12Q 1/02, G01N 33/569

(54) **Bacterial polysaccharide**

(30) Priority: 24.07.1997 GB 9715702
(71) Applicant: UNIVERSITY OF BRISTOL, Bristol BS8 1TH (GB)
(72) Inventor: Stark, Roger M., Bristol BS3 4PH (GB); Millar, Michael R., Bristol BS8 3LN (GB)
(74) Representative: Nash, David Allan

(57) **Abstract**

There is disclosed a purified H. pylori extracellular polysaccharide material (EPM) obtainable by culturing H. pylori in a defined medium as defined in Table 1, and separating the extracellular polysaccharide material from the culture.

## Description

This invention relates to an extracellular polysaccharide material (EPM) of Helicobacter pylori, a method for production of the EPM in vitro, a vaccine against H. pylori which comprises the EPM and a method for inducing active immunization against H. pylori infection using such a vaccine. It also relates to antibodies and therapeutic agents which specifically bind the EPM, and a method for the diagnosis of H. pylori infection which uses the EPM to detect H.pylori-specific antigens.

H. pylori is a Gram-negative, curved, microaerophilic eubacterium. It was first isolated in 1982 from peptic ulcer patients. The organism colonises the mucosal layer of the gastric antrum and is an etiologic agent of type B gastritis, peptic ulcer disease and gastric carcinogenesis in humans. It is estimated that over 50% of the world population is chronically infected by H. pylori.

At present, H. pylori infection is treated by a combination of antibacterial agents, and these have met with some success. However, antibiotic treatment can be expensive, and depends on patient compliance with treatment regimes. In addition antibiotic treatment risks selection for antibiotic-resistant strains of H. pylori. Vaccines based on the urease enzyme have been suggested as have those based on the coccoid protein of H. pylori (WO 97/12910) and a whole cell lysate of H. felis and H. pylori (US 5538729).

There is limited evidence to suggest that H. pylori produces an extracellular polysaccharide (Drouet E.B. *et al* 1993, in "Infection and Immunity" **61** p. 2732-2736).

The present invention is based on the surprising discovery that, when cultured under certain conditions, H. pylori overproduces a hitherto unknown extracellular polysaccharide material (EPM) which can be harvested from the culture vessel and may be used as the basis for a vaccine, or to produce an H. pylori-specific antibody, or as the basis for diagnostic tests for H. pylori. When H. pylori is cultured under standard conditions, this EPM is not detected and so has not previously been made available. Quite unpredictably, the method used in the present invention makes the EPM available in significant quantities for the first time.

Thus, according to a first aspect of the present invention there is provided an H. pylori extracellular polysaccharide material obtainable by culturing H. pylori in a medium as defined in Table 1 below, and separating the extracellular polysaccharide material from the culture.

As mentioned above, it has been found that the EPM is over-produced when an H. pylori strain is cultured in a specific medium as defined in Table 1. Without wishing to be bound by theory, it is believed that the "low" nutrient composition (i.e. low concentration of amino acids and sugars) of the defined medium of Table 1 when compared to "normal" culture media (such as Brucella broth used in the example below) may be important, and in particular the higher carbon to nitrogen ratio in the defined medium of Table 1 to that found in conventional media. It will thus be apparent that the skilled person will be able to adjust the defined medium of Table 1 and still retain a medium which gives over-production of the EPM.

The present invention also extends to the EPM when produced by methods other than culturing H. pylori in the defined medium of Table 1. For example, synthetic EPM may be made using a method such as solid phase synthesis. Alternatively, the gene or genes coding for proteins which organise the production of the EPM may be isolated and cloned into suitable expression vector(s), which may be used to transform a suitable host.

The present invention also extends to natural or synthetic fragments of the EPM containing at least one of the antigenic epitopes of the EPM. In this respect, it is possible that the EPM may be made from multiple repeats of similar or identical short units. The antigenic epitope is the portion (or portions) of the EPM which is bound by a given antibody produced in response to immunization of a mammalian subject with EPM (optionally together with a suitable carrier). Such active fragments can be obtained by partial hydrolysis by chemical or enzymatic means.

The invention also extends to derivatives and/or modifications of the whole EPM and EPM fragments, and which retain the original antigenic activity. For example, when the structure of the antigenic epitope is known, it may be possible to include this epitope in a synthetic molecule, which would have advantages over the EPM or its fragments. For example the synthetic molecule may be easier to produce, may have reduced toxicity or may have increased immunogenicity when compared with the EPM.

As used herein, the term "antigenic activity" is intended to mean the capacity to bind the antigen-binding site of the same antibody that binds an antigenic epitope of the EPM.

When the EPM is obtained by culturing a strain of H. pylori in a defined medium, the EPM may be separated from the culture by methods known in the art, for example centrifugation followed by extraction in phenol/water. If necessary, the EPM may be further purified by a number of known techniques including chromatography using a lectin or an antibody. The object of the separation from the culture medium and any further purification steps is to remove components of the culture medium associated with the EPM, as well as any contaminating proteins and byproducts of the separation stages.

The EPM is relatively insoluble in water, dilute acid, dilute alkali, chloroform, toluene and acetone and is relatively soluble in a phenol/water mixture.

Ideally, in order to produce substantial quantities of the EPM, H. pylori is established in continuous culture, for example in a chemostat. During culture, the medium is continually replenished with quantities of the defined medium specified in Table 1 below.

It is presently believed that all known strains of H. pylori can be employed to obtain the EPM. A specific strain which may be used in the invention to produce the EPM is NCTC 11637.

According to a second aspect of the present invention, there is provided a method for the production of a purified Helicobacter extracellular polysaccharide material which comprises the steps of;
(i) culturing a Helicobacter species in a low nutrient medium; and
(ii) separating the EPM from the culture medium. The low nutrient medium may, for example, be the medium as defined in Table 1 or variants thereof which give the desired overproduction.

The Helicobacter species is preferably a H. Pylori strain. However, the method is also applicable for other species H. pylori, such as H. felis.

According to a third aspect of the present invention there is provided a vaccine for use in vaccinating a subject against H. pylori infection, comprising the purified EPM of the first aspect of this invention, a fragment comprising an antigenic epitope thereof, a derivative/modification of the purified EPM retaining H. pylori antigenic activity or a derivative/modification of the EPM fragment retaining H. pylori antigenic activity.

In order to increase its immunogenicity, the EPM (or fragment or derivative/modification thereof) may be conjugated with a carrier protein or the vaccine may also comprise an adjuvant. Suitable known carrier proteins are toxins and toxoids, such as cholera toxin, tetanus or diphtheria toxoid, and outer membrane proteins such as adhesins.

There are some data available suggesting that the protein and the carbohydrate parts of conjugate vaccines act as independent immunogens. Therefore, the choice of the protein component becomes more important in seeking to enhance immunogenicity. It would be more desirable to have an immunogenic protein or polypeptide derived from H. pylori than a "non-sense" protein. Recently a novel 60kDa antigenic protein obtainable from the coccoid from of H. Pylori has been described (GB 9519865.1), and this would be a candidate for the protein component. Another candidate protein is the urease enzyme which has been implicated as a virulence factor for H. Pylori.

Commonly used adjuvants include inorganic salts such as aluminium hydroxide, aluminium phosphate, calcium phosphate and beryllium hydroxide. Alternatively, whole bacteria, bacterial lysates or bacterial products can be used. Non-pathogenic bacterial strains can used such as B. subtilis, B. punilis or L. plantarum. Alternatively, it is possible to use killed or attenuated bacteria of any strain, notably H. pylori itself. Other adjuvants are currently under development for human use. These include agents which enhance the delivery of the antigen to cells of the immune system such as liposomes and immune stimulatory complexes (ISCOMs) and agents such as cytokines which act as natural mediators of the immune response, for example IL-1, IL-2 and IFN_{γ}.

According to a fourth aspect of the present invention there is provided a method for inducing active immunisation against systemic infection by the pathogen H. Pylori which comprises the step of administering an immunogenic amount of a vaccine as defined above to a mammalian patient.

The vaccine may be administered by any suitable route.

According to a fifth aspect of the present invention there is provided an antibody to H. Pylori which binds specifically to the EPM or fragment thereof. The antibody may be produced by immunization with the EPM or a fragment thereof, or by using a phage display library. The antibody may be used in a number of applications, for example as a diagnostic probe, or as a therapeutic agent.

According to a sixth aspect of the present invention there is provided a therapeutic agent which binds specifically to the EPM. The therapeutic agent may be an antibody or a protein which comprises the antigen-binding site of the antibody molecule.

According to a seventh aspect of the present invention there is provided a method of diagnosis of H. pylori infection which comprises the step of using the EPM to detect antibodies against H. Pylori. The presence of antibodies against H. pylori can be detected in a test sample, such as a blood sample, by performing a binding assay with the EPM. Commonly used binding assays include radioimmunoassays and enzyme-linked immunosorbent assays (ELISA).

The invention will now be described, by way of reference only, to the following example.

### EXAMPLE 1

**a) Bacterial strains.** Type strain H. pylori (NCTC 11637, 11638 & 11916) were sub-cultured every 3-5 days on to Columbia agar base (CM 331, Unipath, Basingstoke, UK) supplemented with 5% horse blood. Plates were incubated in a microaerophilic environment at 37°C. For all experiments NCTC 11637 was used unless otherwise stated.
**b) Batch Culture.** H. pylori harvested from plate cultures was used to inoculate 250ml Brucella broth (#0495-17-3 Difco Ltd, Detroit, USA) containing B-cyclodextrin 0.2% (C-4767, Sigma Chemical Co., Poole, UK) and H. pylori-selective supplement (SR147E, Unipath) to give just visible turbidity (ca. 10⁷ cfu/ml). Cultures were sparged with a mixture of O₂ 5%, CO₂ 10% and N₂ 85% at a rate of 75ml/min and agitated in an orbital shaker at 100 rpm. Broth cultures were harvested at 72-96 hours and the bacterial cells concentrated by centrifugation.
**c) Growth in continuous culture.** H. pylori was established in continuous culture as previously described using filter sterilised Brucella broth supplemented with foetal calf serum 1%. After six days the undefined Brucella broth was replaced with a defined medium (Table I) with constituents based on the published reports of the nutritional and growth supplement requirements of H. pylori. This medium was filter sterilised. The defined medium was added at a continuous flow rate to give a net dilution rate (D) of 0.03 h-¹.
**d) Estimation of culture biomass and ammonia.** At intervals, samples of the chemostat culture were removed. The biomass was estimated by measuring the OD₅₄₀. Ammonia concentrations samples was determined using the Berthelot (phenol-hypochlorite) method.
**e) Chemostat purity.** The purity of the chemostat broth culture was checked each day by inoculation of Columbia blood agar which was then incubated aerobically and microaerophilically for up to five days.

### Results

In the continuous culture experiments, growth in undefined-brucella broth with foetal calf serum 1% was followed by the transition to a fully defined medium. Throughout the experiment, the biomass, ammonia concentration and pH of the culture were measured at intervals. Growth in the undefined medium did not require the addition of acid or base, since the pH remained approximately constant within the set limits (6.5-7.2). Growth in the defined medium (started at day 6) did require the addition of acid and base to maintain the pH. The transition from undefined to defined media was carried out using a dilution rate of 0.03 h-¹ (21ml/h with 700ml culture volume). After 18 days of defined medium addition, the residual brucella broth concentration was therefore less than 0.1%.

Twelve days after inoculation of the chemostat an EPM was observed to have formed on the vessel wall. This was most marked at the gas/liquid interface where foaming occurred. Build-up of the EPM led to inconsistent biomass determinations by optical density as occasionally sections of EPM became dislodged. Eventually the overflow (waste) line of the chemostat became blocked with build up of EPM 24 days after inoculation. Regular purity checks confirmed the culture to be H. pylori.

The EPM stained with a periodic acid/Schiff reaction, indicating that it was a polysaccharide. It was soluble in phenol-water but insoluble in alcohol, chloroform, toluene, acetone, dilute acid or dilute alkali.

It has been demonstrated, using a Western blotting technique, that antibodies to the EPM are present in the sera of patients with H. pylori infection. This shows that the EPM is produced in vivo.

**TABLE 1**

| Constituents of defined medium for growth of H. pylori in continuous culture | | | | |
|---|---|---|---|---|
| | | | | |
| L-alanine | 150mg/1 | (1.68mM) | Thiamine | 0.03mg/1 |
| L-arginine | 150 | (0.71) | Hypoxanthine | 10 |
| L-asparagine | 70 | (0.52) | Nitrapyrin | 0.5 |
| L-aspartic acid | 150 | (1.11) | Adenine | 10 |
| L-cysteine | 70 | (0.45) | Uracil | 10 |
| L-cystine | 70 | (0.29) | Guanine | 10 |
| L-glutamic acid | 150 | (1.02) | Xanthine | 10 |
| L-glutamine | 150 | (1.02) | | |
| Glycine | 70 | (0.93) | NaC1 | 4500 |
| L-histidine.HC1 | 70 | (0.45) | KCl | 4000 |
| L-isoleucine | 150 | (1.14) | K₂HPO₄ | 500 |
| L-leucine | 150 | (1.14) | MgSO₄.7H₂O | 100 |
| L-lysine | 150 | (1.02) | CaCl₂ | 20 |
| L-methionine | 70 | (0.47) | MnCl₂ | 10 |
| L-phenylalanine | 150 | (0.91) | CoCl₂ | 0.2 |
| L-proline | 150 | (1.30) | CuCl₂ | 0.01 |
| L-serine | 150 | (1.42) | ZnSO₄ | 0.1 |
| L-threonine | 150 | (1.26) | NICl₂ | 0.02 |
| L-trypotophan | 70 | (0.34) | Na₂MoO₄ | 0.03 |
| L-tyrosine | 150 | (0.82) | FeSO₄^{∗} | 2.0 |
| L-caline | 150 | (1.28) | H₃BO₄ | 0.3 |
| β-cyclodextrin | 1000 | | Glucose | 2000 |

| | | | | |
|---|---|---|---|---|
| ∗ or Fe(III) salt, e.g. ferric pyrophosphate | | | | |

## Claims

1. A purified H. pylori extracellular polysaccharide material (EPM) obtainable by culturing H. pylori in a defined medium as defined in Table 1, and separating the extracellular polysaccharide material from the culture.

2. A fragment of the extracellular polysaccharide material of claim 1 which comprises one or more antigenic epitopes of the extracellular polysaccharide material.

3. A method for the production of the purified extracellular polysaccharide material of claim 1 which method comprises the steps of;
(i) culturing a Helicobacter species in a low nutrient medium;
(ii) separating the EPM from the culture medium; and
(iii) purifying the EPM.

4. A method according to claim 3 in which the low nutrient medium is the medium as defined in Table 1.

5. A method according to claim 3 or 4 in which the Helicobacter species is an H. pylori strain.

6. A vaccine comprising the purified EPM of claim 1, or an antigenic fragment thereof.

7. A method for inducing active immunisation against systemic infection by the pathogen H. Pylori which comprises the step of administering an immunogenic amount of a vaccine according to claim 6 to a mammalian patient.

8. An antibody to H. Pylori which binds specifically to the EPM of claim 1.

9. A therapeutic agent which binds specifically to the EPM of claim 1.

10. A method of diagnosis of H. pylori infection which comprises the step of using the EPM of claim 1 to detect antibodies against H. Pylori.
